# EUROPÄISCHE PATENTSCHRIFT

(11) **EP 2 042 483 B1**
(45) Veröffentlichungstag und Bekanntmachung des Hinweises auf die Patenterteilung: **28.08.2013**
(21) Anmeldenummer: 08164473.4
(22) Anmeldetag: 17.09.2008
(51) Int. Cl.: C07C 241/02, C07C 243/14

(54) **Verfahren zur herstellung von monomethylhydrazin**
Method for manufacturing monomethyl hydrazine
Procédé de fabrication de monométhylhydrazine

(30) Priorität: 28.09.2007 DE 102007046467
(43) Veröffentlichungstag der Anmeldung: 01.04.2009
(73) Patentinhaber: Saltigo GmbH, 40764 Langenfeld (DE)
(72) Erfinder: Böger, Uwe, Herr, 51375, Leverkusen (DE); Notheis, Ulrich, Herr, 41539, Dormagen (DE); Siekmann, Mathias, Herr, 51061, Köln (DE)
(74) Vertreter: Pettrich, Klaus-Günter

(56) Entgegenhaltungen:
- DE-A1- 3 148 971
- FR-A- 1 260 474
- DATABASE WPI Week 198343 Thomson Scientific, London, GB; AN 1983-798677 XP002512438 -& JP 58 157751 A (NIPPON HYDRAZINE KO) 19. September 1983 (1983-09-19)
- RICHARD L. HINMAN: "Base Strengths of Some Alkylhydrazines" JOURNAL OF ORGANIC CHEMISTRY, Bd. 23, 1958, Seiten 1587-1588, XP002512432
- '1-methylimidazole', [Online] 17 Mai 2011, Wikipedia Gefunden im Internet: <URL:http://en.wikipedia.org/wiki/1-methyli midazole> [gefunden am 2011-07-21]
- 'Tripropylamin', [Online] 25 April 2011, Wikipedia Gefunden im Internet: <URL:http://de.wikipedia.org/wiki/Tripropyl amin> [gefunden am 2011-07-25]

## Beschreibung

Die vorliegende Erfindung betrifft ein Verfahren zur Herstellung von Monomethylhydrazin.

Monomethylhydrazin, im folgenden MMH genannt, ist ein wertvolles Ausgangsmaterial für die Herstellung von Pflanzenschutzmitteln und Pharmazeutika. Es wird aber auch in der Weltraum-und Raketentechnik als Treibstoff verwendet.

In technischem Maßstab wird MMH überwiegend durch einen modifizierten Raschig-Prozess hergestellt. Dabei wird in einem ersten Schritt aus Ammoniak und Natriumhypochlorit Chloramin hergestellt, das dann in einem zweiten Schritt mit Methylamin zum MMH umgesetzt wird.

Alternativ kann Methylharnstoff mit Natriumhydroxid und Natriumhypochlorit zu MMH umgesetzt werden. Beide Verfahren führen zu einer sehr niedrigen Produktkonzentration, so dass energieintensive Trennschritte mit aufwändigen Anlagen für die Aufkonzentration und Aufreinigung benötigt werden.

Weiterhin ist ein Verfahren zur Nitrosierung von Methylamin und Hydrierung des entstandenen Nitrosamins zum Methylhydrazin bekannt. Wegen der Karzinogenität der Zwischenstufe wird das Verfahren jedoch nicht industriell angewandt.

Weiterhin kann MMH durch Alkylierung von Hydrazin oder Hydrazinverbindungen hergestellt werden. Auf diesem Gebiet sind verschiedene Verfahren beschrieben worden.

Als technisch verfügbare Alkylierungsmittel sind beispielsweise Dimethylsulfat, Methylchlorid, Methanol/Methylchlorid, Methanol in Gegenwart einer HCl-Quelle und Trimethylaniliniumhalogenide beschrieben.

Generell besteht bei der Alkylierung von Hydrazin die Schwierigkeit, dass die Reaktion nicht auf der Stufe der Monoalkylierung stehen bleibt, sondern dass man je nach Reaktionsbedingungen und Grad der Alkylierung Gemische aus nicht umgesetztem Hydrazin, MMH, und mehrfach alkylierten Hydrazinen wie symmetrischem Dimethylhydrazin (SDMH), unsymmetrischem Dimethylhydrazin (UDMH) und Trimethylhydrazin (TMH) und Hydraziniumverbindungen erhält, die gegebenfalls schwierig aufzutrennen sind.

Alkyliert man nicht das freie Hydrazin sondern ein Hydrazinium-Salz als Hydrazinverbindung sind zwar höhere Temperaturen erforderlich, aber die Selektivität zum monoalkylierten Produkt ist bei gleichem Umsatz höher als bei der Alkylierung des freien Hydrazins.

Typischerweise wird daher nur ein Teilumsatz bezüglich Hydrazin oder Hydrazinverbindung eingestellt, um eine hohe Selektivität zum einfach alkylierten Produkt zu erzielen. Für die Wirtschaftlichkeit ist es dann besonders wichtig nicht umgesetztes Hydrazin effizient abzutrennen und zurückzuführen.

Nach der Alkylierung bildet die Abgangsgruppe des Alkylierungsmittels -ggf. unter Freisetzung eines weiteren ungeladenen Moleküls - mit den Hydrazinen in der Reaktionsmischung ein Salz. Aus diesem Salz müssen die gebildeten Alkylhydrazine und/oder das Ausgangshydrazin in der Aufarbeitung freigesetzt und abgetrennt werden.

MMH und Hydrazin bilden mit Wasser binäre Hochsiederazeotrope. Das Azeotrop MMH/Wasser enthält ca. 3 Mol Wasser auf ein Mol MMH und siedet bei 102-106°C. Hydrazin bildet ein äquimolares Azeotrop mit Wasser, das bei 120-121°C siedet. Will man daher die Hydrazine nach ihrer Freisetzung mit Alkalihydroxid vom neu gebildeten Salz abtrennen, geht je nach Zusammensetzung erst Wasser, dann das Azeotrop MMH/Wasser, dann das Azeotrop Hydrazin/Wasser über und das Salz bleibt als Feststoff zurück. In der Technik erfordert dies spezielle aufwändige Apparaturen. Weiterhin besteht in feststoffhaltigen Mischungen die Gefahr der Krustenbildung und der lokalen Überhitzung, was aus Gründen der thermischen Stabilität von Hydrazin vermieden werden muss.

US-A 2 954 283 beschreibt die Herstellung einer Mischung überwiegend aus MMH und UDMH durch Umsetzung von Dimethylsulfat mit Hydrazin oder Hydraziniumsulfat. Dimethylsulfat als Alkylierungsmittel hat den Nachteil, dass die beiden Methylgruppen nicht vollständig ausgenutzt werden können. Die bei unvollständiger Ausnutzung entstehende Methylschwefelsäure ist biologisch sehr schlecht abbaubar und muss daher in einem zusätzlichen Schritt hydrolisiert werden.

DE-A 31 48 971 beschreibt ein Verfahren zur Herstellung von Monomethylhydrazin, bei dem Hydrazinhydrochlorid mit Methanol in Gegenwart von Hydrazindihydrochlorid oder Methylchlorid zu Monomethylhydrazinhydrochlorid umgesetzt wird. Dabei werden Umsätze an Hydrazinmonohydrochlorid von maximal 30% beschrieben. Die Produktmischung wird anschließend destillativ von Methanol und Methylchlorid befreit, das zurückgeführt werden kann.

Gegebenenfalls enthaltenes Wasser wird ebenfalls destillativ entfernt. Danach wird dem Rückstand Methanol zugesetzt, wobei kristallines Hydrazinmonohydrochlorid als Feststoff ausfällt und abgetrennt wird. Dieses Hydrazinhydrochlorid kann in die Reaktion zurückgeführt werden. Die nach der Abtrennung von Hydrazinhydrochlorid verbliebene Mischung wird mit wässriger Alkalilösung versetzt. Dadurch wird das Methylhydrazin freigesetzt und kann destillativ isoliert und gereinigt werden.

Dieses Verfahren hat jedoch den Nachteil, dass nur bei relativ kleinen Umsätzen gute Selektivitäten zu MMH erzielt werden, wie eigene Versuche zeigen.

Ferner erfordert das Verfahren die destillative Abtrennung von Methanol und Wasser aus der Reaktionsmischung und anschließend die Zugabe von frischem Methanol.

Hydrazinhydrochlorid ist teilweise in Methanol löslich, so dass nach Neutralisation der Mutterlauge mit Natronlauge sowohl Monomethylhydrazin als auch Hydrazin in den Rektifikationsschritt gelangen. Das Hydrazin muss hier ebenfalls abgetrennt und zurückgeführt werden. Der beschriebene Rektifikationsschritt hat darüberhinaus den massiven Nachteil, dass nach der Destillation der freigesetzten Hydrazine ein fester Rückstand von NaCl im Sumpf zurückbleibt. Diese Destillation kann daher nur in speziellen Apparaturen geschehen und ist angesichts der Zersetzungsneigung der Hydrazine auch aus Sicherheitsgründen kritisch.

Ohne Rückführung des Hydrazinhydrochlorids ist das Verfahren wegen der geringen Ausbeute bezogen auf eingesetztes Hydrazin unwirtschaftlich. Somit müssen große Mengen nicht umgesetzten Hydrazinhydrochlorids in einer komplizierten Aufarbeitung unter Handhabung von Feststoffen wieder zurückgeführt werden.

JP-A-58157751 beschreibt einen alternativen Prozess zur Gewinnung von Monomethylhydrazin aus monomethylhydrazinhydrochlorid-haltigen Produktmischungen, die durch Umsetzung von Hydrazinhydrochlorid mit Methanol in Gegenwart von Hydrazindihydrochlorid oder Methylchlorid hergestellt wurden.

Dabei wird die Reaktionsmischung durch destillative Abtrennung von Methanol und Wasser aufkonzentriert und mit einem 1,2 bis 2 fachen Überschuss an Hydrazinhydrat (HyHy) bezogen auf vorhandenes Hydrochlorid versetzt. Diese Mischung, die 2,2 - 3,0 mol Hydrazine bezogen auf vorhandenes Hydrochlorid enthält, wird dann destilliert, um das niedriger als Hydrazin siedende Methylhydrazin freizusetzen. Auch ein Teil des zugesetzten Hydrazins kann zurückgewonnen werden. Die maximale Temperatur der Reaktionsmischung bei der Destillation soll unter 120°C liegen, was bei einem Siedepunkt von Hydrazinhydrat von 120°C eine Vakuumdestillation erfordert.

Dabei müssen jedoch Leckagen in der Apparatur, die zu Lufteintritt und Reaktion der Hydrazine mit Luftsauerstoff führen können, ausgeschlossen werden, was für eine technische Anlage aufwändige Sicherheitsmaßnahmen erforderlich macht.

Das beschriebene Vorgehen ist daher als technisches Verfahren unvorteilhaft.

JP-A-60237059 beschreibt ein Verfahren zur Herstellung von Monoalkylhydrazinen, in dem Hydrazin, Methanol und eine Halogenwasserstoffsäure in Gegenwart einer phosphorhaltigen Sauerstoffsäure zu Monomethylhydrazin umgesetzt werden. Die Reaktionsmischung wird im Vakuum aufkonzentriert und mit 50%iger Natronlauge neutralisiert. Dabei fällt ein anorganischer Feststoff aus. Dieser Feststoff wird durch Filtration abgetrennt, nochmals gewaschen und die vereinigten Flüssigphasen werden fraktioniert destilliert. Nach der Destillation bleibt ein anorganisches Salz zurück, das grosse Teile des nicht umgesetzten Hydrazins enthält. Eine Rückführung des Hydrazins wird nicht beschrieben.

Wegen der geringen Ausbeute bezogen auf eingesetztes Hydrazin ist das Verfahren unwirtschaftlich.

DE-A 30 26 771 beschreibt ein Verfahren zur Herstellung von MMH und unsymmetrischem Dimethylhydrazin (UDMH), dadurch gekennzeichnet, dass man Trimethylaniliniumhalogenid mit Hydrazin bzw. MMH umsetzt. Dabei wird ein Überschuss 5 bis 12:1 von Hydrazinen zu Trimethylaniliniumhalogenid verwendet. Entstandenes Dimethylanilin wird als organische Phase durch Phasenscheidung abgetrennt und MMH und UDMH durch Destillation der wässrigen Phase gewonnen.

Dieses Verfahren hat den Nachteil, dass Dimethylanilin zurückgeführt und in einem zusätzlichen Schritt mit Methylchlorid zum Alkylierungsmittel umgesetzt werden muss.

Der vorliegenden Erfindung liegt nun die Aufgabe zugrunde, ein Verfahren zur Herstellung von Monomethylhydrazin bereitzustellen, das in konventionellen Mehrzweckapparaten ohne aufwändige Entwicklung aus gut verfügbaren Chemikalien durchführbar ist.

Die Erfindung betrifft ein Verfahren zur Herstellung von Monomethylhydrazin durch Methylierung von Hydrazin oder Hydraziniumhydro- oder -dihydrochlorid mit Methylchlorid und/oder einem Methanol/HCl-Gemisch, dadurch gekennzeichnet, dass man die bei der Methylierung entstandene Reaktionsmischung mit einer geeigneten organischen Base mit einem pkₐ-Wert größer 7,0 und einem Siedepunkt über 120°C umsetzt, wobei die organische Base Triethanolamin, Diethanolamin, Monoethanolamin oder eine Mischung daraus ist und Monomethylhydrazin in einer Leichtsiederfraktion aus der Reaktionsmischung destillativ abtrennt und die Leichtsiederfraktion gegebenenfalls einer weiteren Destillation unterzieht.

Die Reaktionsmischung kann erfindungsgemäß auf verschiedenen Wegen erhalten werden.

Einmal kann die Reaktionsmischung durch Umsetzen von Hydrazinhydrochlorid mit Methanol in Gegenwart eines Überschusses an HCl (bezogen auf Hydrazin) hergestellt werden, wie etwa in DE-A 3148971 beschrieben. Der HCl-Überschuss kann durch Zusatz von Hydrazindihydrochlorid, HCl oder Methylchlorid erzeugt werden. Ohne den Überschuss läuft die Reaktion nicht ab. Bevorzugt werden 5 - 20% HCl-Überschuss eingestellt.

In einer Vorgehensweise kann Hydrazinhydrat in einem Reaktor vorgelegt werden und gasförmige HCl zudosiert werden. HCl kann auch als wässrige Salzsäure zugegeben werden. Bevorzugt ist die Zugabe von HCl als Gas. Die Wassermenge bezogen auf Hydrazin liegt bei 1:1 (HyHy mit gasförmiger HCl) bis etwa 10:1 (verdünntere Hydrazinlösung mit wässriger HCl). Bevorzugt sind Wasseranteile von 1:1 bis 5,5:1, besonders bevorzugt 1:1 bis 3: 1.

Die Umsetzung findet erfindungsgemäß bei Temperaturen von 90 bis 150°C, vorzugsweise 100 bis 130°C statt. Der Druck in der Reaktion hängt von der Reaktionstemperatur, den Mengen an Methanol und Wasser und der Menge an überschüssiger HCl ab.

Ein Druck von 2 bis 15 bar, besonders von 2 bis 5 bar, wird typischerweise bevorzugt.

In einer weiteren Ausführungsform wird Hydrazinhydrochlorid mit dem Überschuss an HCl vorgelegt und auf Temperatur gebracht. Dann wird Methanol zudosiert, bis der gewünschte Druck erreicht ist und die Dosiergeschwindigkeit von Methanol so eingestellt, dass der Druck im System konstant bleibt. Nach Dosierende fällt der Druck, und man wartet ab, bis er sich nicht mehr ändert.

Die molare Menge an Methanol kann zwischen 50% und 600% bezogen auf Hydrazin liegen und komplett oder nur teilweise umgesetzt werden. Je höher die umgesetzte Menge an Methanol, desto geringer ist die Selektivität zu MMH. Die Ausbeute steigt jedoch bis zu einem Verhältnis von etwa 1:1 weiter an. Bevorzugt wählt man die Methanolmenge und die Dauer der Reaktion so, dass am Ende der Umsetzung alles zugegebene Methanol abreagiert ist. In diesem Fall sind Methanolmengen von 0,5:1 bis 1:1 bezogen auf Hydrazin bevorzugt.

Zum anderen kann die Reaktionsmischung durch Umsetzung von Hydrazin mit Methylchlorid erhalten werden. Hydrazin kann als Hydrazinhydrat oder als verdünntere wässrige Lösung eingesetzt werden. Bevorzugt ist die Verwendung von 100%igem Hydrazinhydrat.

Methylchlorid kann als Gas oder verdichtet als Flüssigkeit unter entsprechendem Druck eingesetzt werden. Die Reaktion kann batchweise unter Zudosieren von Methylchlorid oder kontinuierlich ausgeführt werden.

Die Reaktionstemperaturen liegen zwischen 40 und 100°C, bevorzugt zwischen 60 und 90°C. Der Druck hängt von der Menge an Methylchlorid und der Reaktionstemperatur ab. Durch Vorgabe der Temperatur und der Methylchloridmenge bzw. der Dosiergeschwindigkeit des Methylchlorids kann der Druck auf die Anforderungen der gewählten Apparatur angepasst werden. Typischerweise liegt er im Bereich zwischen 1 und 10 bar, bevorzugt zwischen 1 und 6 bar.

Da die Selektivität zu MMH bei dieser Ausführungsform stark vom Umsatz abhängt, ist es vorteilhaft, den Umsatz bezüglich Hydrazin auf 50%, bevorzugt 30% zu beschränken.

Die so hergestellten Reaktionsmischungen werden wie folgt weiterverarbeitet:

Sollte die Reaktionsmischung noch leichtflüchtige Verbindungen wie Methanol oder Methylchlorid enthalten, werden diese vor der Freisetzung der Hydrazine destillativ abgetrennt.

Die oben beschriebene Reaktionsmischung wird mit einer organischen Base im Verhältnis 1:1,05 bis 1:3, bevorzugt 1:1,05 bis 1:2, besonders bevorzugt 1:1,05 bis 1:1,3 umgesetzt. Flüchtige Komponenten, die in der erfindungsgemäßen Destillation mindestens teilweise über Kopf abgenommen werden, sind Hydrazin, MMH, UDMH, SDMH, TMH und Wasser.

Im erfindungsgemäßen Verfahren eingesetzte organische Basen weisen folgende Kennzeichen auf:
Ihr pKa-Wert größer 7,0 liegt über dem von Hydrazin, bevorzugt über pKa = 8,0 besonders bevorzugt über pKa = 9,0. Der pKa von Hydrazin und Monomethylhydrazin ist in Tabelle 1 von Richard L Hinman, "Base Strengths of Some Alkylhydrazines", Journal of Organic Chemistry, 23, 1958, Seiten 1587-1588 beschrieben.
Ihr Normalsiedepunkt liegt über dem Siedepunkt des Hydrazin/Wasser Azeotrops, also über 120°C, aber unter 250°C, bevorzugt bei Umgebungsdruck über 140°C und unter 220°C, besonders bevorzugt bei Umgebungsdruck zwischen 160°C und 200°C.

Sie bilden mit Wasser keine Azeotrope, die unterhalb des MMH/Wasser Azeotrops sieden, besonders bevorzugt bilden sie keine Azeotrope mit Wasser.

Ihre Hydrochloride sind bei Destillationsbedingungen flüssig.

Bevorzugt sind Monoethanolamin, Diethanolamin und Mischungen, wie sie bei der technischen Synthese dieser Verbindungen anfallen. Ganz besonders bevorzugt ist Monoethanolamin.

Die Mengen an organischer Base, die mit über Kopf destillieren, hängen in an sich bekannter Weise von der Trennleistung der verwendeten Kolonne und dem eingestellten Rücklaufverhältnis ab. Bevorzugt wird eine Kolonne mit 1-20 theoretischen Stufen verwendet und ein Rücklaufverhältnis von 0,1:1 bis 10:1 eingestellt. Je besser die Rückhaltung der organischen Base im Sumpf ist, umso kleiner kann der Überschuss an organischer Base gewählt werden.

Die Destillation kann grundsätzlich batchweise, semibatchweise oder kontinuierlich betrieben werden. Bevorzugt sind semibatch- oder kontinuierliche Fahrweisen. Die Destillation kann bei Umgebungsdruck oder bei Unterdruck ausgeführt werden. Bevorzugt ist die Destillation bei Normaldruck.

In einer bevorzugten semibatch-Ausführungsform wird die organische Base vorgelegt und auf eine Temperatur zwischen 130-170°C gebracht, und die methanolfreie Reaktionsmischung in die Base derart zudosiert, dass Methylhydrazin vollständig über Kopf abdestilliert. Im Sumpf verbleiben das Hydrochlorid der Base und die Base. Während der Destillation zersetzt sich teilweise Hydrazin. Die Zusammensetzung des Kopfproduktes kann über die Kopftemperatur kontrolliert und über das Rücklaufverhältnis beeinflusst werden. Die Kopftemperatur liegt bei Umgebungsdruck zwischen 100°C und der Siedetemperatur der Base, bevorzugt zwischen 100°C und 130°C. Die Dosiergeschwindigkeit wird so eingestellt, dass keine sicherheitskritische Anreicherung von Hydrazinen im Sumpf erfolgt.

In einer anderen bevorzugten semibatch-Ausführungsform wird die Reaktionsmischung so zudosiert, dass alle flüchtigen Komponenten unmittelbar über Kopf abdestillieren.

In einer bevorzugten kontinuierlichen Ausführungsform wird die Reaktionsmischung mit der organischen Base gemischt und dann kontinuierlich in einem geeigneten Apparat verdampft. Geeignete Apparate sind Apparate für eine kontinuierliche Destillation, beispielsweise Dünnschichtverdampfer, Kurzweg- oder Fallfilmverdampfer. Temperatur und Verweilzeit im Verdampfer werden so eingestellt, dass MMH vollständig über Kopf abdestilliert. Der Verdampfer kann im Verbund mit einer Trennkolonne (z.B. Packungs-, Füllkörper- oder Bodenkolonne) betrieben werden.

Gegebenenfalls kann ein Teil der organischen Base durch ein Alkalihydroxid, bevorzugt NaOH oder wässrige Natronlauge, so ersetzt werden, dass der Sumpf der Destillation im Ablauf noch förderbar bleibt.

Wenn die flüchtigen Verbindungen abgetrennt sind, werden die restlichen Mengen an freier organischer Base aus dem Sumpf abgetrennt und zurückgeführt. Dabei werden letzte im Sumpf befindliche Spuren an Hydrazinen thermisch zersetzt.

Ist die organische Base ein Alkanolamin wie Monoethanolamin, so enthält der dann verbleibende Sumpf nur biologisch gut abbaubare organische Komponenten und kann -gegebenenfalls nach Neutralisation mit einer Base wie Natronlauge oder Kalkmilch- als Abwasser in eine Kläranlage entsorgt werden.

Bildet die organische Base nach Freisetzen mit einer starken wässrigen anorganischen Base eine zweite flüssige Phase neben der wässrigen Salzlösung aus, so kann diese abgetrennt und wieder zurückgeführt werden.

Gegebenenfalls kann die erhaltene Leichtsiederfraktion, die MMH enthält, einer weiteren Destillation (Feindestillation) zur Abtrennung von UDMH, SDMH und weiterer eventueller Nebenprodukte zugeführt werden.

In einer besonders bevorzugten Ausführungsform wird 100%iges Hydrazinhydrat in einem Rohrreaktor oder einer Kesselkaskade mit bis zu vier Kesseln kontinuierlich mit Methylchlorid umgesetzt. Die Reaktionsmischung wird mit der organischen Base umgesetzt und die flüchtigen Komponenten werden kontinuierlich über Kopf abgetrennt. Die über Kopf abgetrennten Alkylhydrazine MMH und UDMH werden in einem zweiten Destillationsschritt vom Hydrazinhydrat abgetrennt, das wieder in die Reaktion zurückgeführt wird. Anschließend wird die Mischung aus UDMH und MMH in bekannter Weise weiter zu verkaufsfähigem MMH und UDMH destilliert. Die Feindestillation kann kontinuierlich oder auch batchweise durchgeführt werden.

### Beispiele

### Allgemeines

Die Hydrazinanalysen wurden mittels Kapillarelektrophorese durchgeführt.

### Vergleichsbeispiel 1 (nicht erfindungsgemäß) Semibatch-Destillation mit Tri-n-propylamin (NPr₃) als Base

In einem 2 L Vierhalskolben mit mechanischem Rührer und einer aufgesetzten 20 cm langen Silbermantelkolonne mit einem Durchmesser von 25 mm, die mit 4 x 4 mm Raschigringen gefüllt und mit automatischem Rücklaufteiler versehen war, wurden 525 g (3,6 mol) Tripropylamin vorgelegt und auf 160°C erhitzt. In 1 Stunde und 35 Minuten pumpte man mit einer Schlauchpumpe 200 g einer wässrigen Reaktionsmischung, die 19 g Hydrazin, 32 g MMH, 22 g mehrfach alkylierte Hydrazine und 66 g HCl enthielt, in das siedende Tripropylamin ein. 10 Minuten nach Dosierstart schaltete man den Rücklaufteiler von vollständigem Rücklauf auf ein Rücklaufverhältnis R:E von 4:1. Innerhalb von 3 Stunden destillierten 172 g einer Mischung über, die lediglich 2 g MMH enthielt (6% Destillationsausbeute).

### Vergleichsbeispiel 2 (nicht erfindungsgemäß) Semibatch-Destillation mit N-Methylimidazol als Base

Es wird vorgegangen wie in Vergleichsbeispiel 1, aber als Amin wurden 470 g N-Methylimidazol vorgelegt und auf 170°C erwärmt. In 1 Stunde und 33 Minuten pumpte man mit einer Schlauchpumpe 308 g einer wässrigen Reaktionsmischung, die 29 g Hydrazin, 50 g MMH, 35 g mehrfach alkylierte Hydrazine und 99 g HCl enthielt, in das siedende N-Methylimidazol ein. 14 Minuten nach Dosierstart schaltete man den Rücklaufteiler von vollständigem Rücklauf auf ein Rücklaufverhältnis R:E von 4:1. Innerhalb von 4 Stunden und 15 Minuten destillierten 142 g einer Mischung über, die lediglich 2,4 g MMH enthielt (5% Destillationsausbeute).

### Beispiel 1: Herstellen einer Rohmischung aus Hydrazinhydrochlorid und Methanol.

Im Wasserbad wurde eine Lösung aus 1269 g Hydrazinhydrochlorid, 102 g Hydrazindihydrochlorid und 349 g Wasser hergestellt und in einen 3 L Stahl-Emaille-Autoklaven mit Rührer und Tauchrohr überführt. Der Autoklav wurde verschlossen und auf 130°C aufgeheizt. Innerhalb von 4 Stunden wurden bei 110°C 560 g Methanol so zugepumpt, dass der Druck 4 bar nicht überstieg. Anschließend wurde für 4 Stunden nachgerührt, der Autoklav auf 60°C abgekühlt, entspannt und entleert. Das Produkt enthielt 9,8 Gew% Hydrazin, 16,9 Gew% MMH, 5,0 Gew% UDMH, 4,4 Gew% SDMH und 1,8 Gew% TMH sowie 32,8 Gew% HCl.

### Beispiel 2: Semibatch-Destillation mit Monoethanolamin (MEA) als Base

In einem 2 L Vierhalskolben mit mechanischem Rührer und einer aufgesetzten 40 cm langen Silbermantelkolonne mit einem Durchmesser von 25 mm, die mit 4 x 4 mm Raschigringen gefüllt und mit automatischem Rücklaufteiler versehen war, wurden 605 g (9,8 mol) Monoethanolamin vorgelegt und auf 170°C erhitzt.

In 2 Stunden und 33 Minuten pumpte man 800 g der in Beispiel 1 hergestellten Lösung ein. Die Kopftemperatur betrug zwischen 103 und 107°C, die Sumpftemperatur 150°C. Das Rücklaufverhältnis betrug 4:1. Nach Dosierende wurde für 2 Stunden und 10 Minuten unter gleichen Bedingungen weiterdestilliert. Es wurde eine Fraktion von 496 g abgenommen, die 26,5 Gew%. MMH (entsprechend 97% des in die Destillation eingesetzten MMHs) sowie 0,4 % Ethanolamin enthielt.

Anschliessend wurde zunächst bei Umgebungsdruck und einer Sumpftemperatur von 170°C weiterdestilliert, dann langsam Vakuum angelegt und im Vakuum bis zu einer Sumpftemperatur von 200°C und 10 mbar Kopfdruck eine zweite Fraktion von 208 g abdestilliert, die 75,2 Gew% Ethanolamin, 11,5 Gew% Hydrazin sowie 1,5 Gew% MMH enthielt, und die in die nächste Destillation eingesetzt werden konnte.

### Beispiel 3: Herstellen einer Rohmischung aus Hydrazin und Methylchlorid.

In einem 1,4 L Emaille-Autoklav wurden 400 g (8 mol) Hydrazinhydrat vorgelegt und der Autoklav auf 80°C erwärmt. Bei dieser Temperatur wurden in 3 Stunden und 15 Minuten 101 g (2 mol) Methylchlorid so zudosiert, dass der Druck 4 bar nicht überstieg. Nach Dosierende wurde für drei Stunden nachgerührt, auf Raumtemperatur abgekühlt, entspannt und das Produkt entnommen. Das Produkt enthielt 42,0 Gew% Hydrazin, 8,9 Gew% Methylhydrazin, 4,8 Gew% UDMH und 12,5 Gew% Chlorid.

### Beispiel 4: Destillation einer Rohmischung über einen Dünnschichtverdampfer bei 140°C

178,8 g des in Beispiel 3 hergestellten Reaktionsproduktes, die 0,63 mol Chlorid enthielten, wurden mit 46,2 g (0,76 mol) Monoethanolamin versetzt und in eine auf 90 °C beheizte Vorlage gefüllt. Aus dieser Vorlage wurde die Mischung in 1 Stunde und 5 Minuten über einen Sambay-Labordünnschichtverdampfer mit einer Heizfläche von 155 cm² gefahren. Die Heizmitteltemperatur betrug 140°C. Man erhält 83 g Destillat und 142 g Sumpfprodukt. Das Destillat enthielt 16,7 Gew% MMH (entsprechend 82% des in die Destillation eingesetzten MMHs).

### Beispiel 5: Destillation einer Rohmischung über einen Dünnschichtverdampfer bei 150°C

287 g des in Beispiel 3 hergestellten Reaktionsproduktes, die 1,01 mol Chlorid enthielten, wurden mit 74 g (1,21 mol) Monoethanolamin versetzt und in eine auf 90 °C beheizte Vorlage gefüllt. Aus dieser Vorlage wurde die Mischung in 1 Stunde und 41 Minuten über den Sambay-Labordünnschichtverdampfer aus Beispiel 4 gefahren. Die Heizmitteltemperatur betrug 150°C. Man erhält 185 g Destillat und 176 g Sumpfprodukt. Das Destillat enthielt 13,5 Gew% MMH (entsprechend 98% des in die Destillation eingesetzten MMHs).

### Beispiel 6: Destillation einer Mischung mit Dünnschichtverdampfer bei 160°C

243 g des in Beispiel 3 hergestellten Reaktionsproduktes, die 0,86 mol Chlorid enthalten, wurden mit 62,7 g (1,03 mol) Monoethanolamin versetzt und in eine auf 90 °C beheizte Vorlage gefüllt. Aus dieser Vorlage wurde die Mischung in 2 Stunden und 55 Minuten über den Sambay-Labordünnschichtverdampfer aus Beispiel 4 gefahren. Man erhielt 181 g Destillat und 125 g Sumpfprodukt. Das Destillat enthielt 11,8 Gew% MMH (entsprechend 99% des in die Destillation eingesetzten MMHs).

## Patentansprüche

1. Verfahren zur Herstellung von Monomethylhydrazin durch Methylierung von Hydrazin oder Hydraziniumhydro- oder -dihydrochlorid mit Methylchlorid und/oder einem Methanol/HCl-Gemisch, **dadurch gekennzeichnet, dass** man die bei der Methylierung entstandene Reaktionsmischung mit einer organischen Base mit einem pKa-Wert größer 7,0 und einem Siedepunkt über 120°C umsetzt, wobei die organische Base Triethanolamin, Diethanolamin, Monoethanolamin oder eine Mischung daraus ist und Monomethylhydrazin in einer Leichtsiederfraktion aus der Reaktionsmischung destillativ abtrennt und diese Leichtsiederfraktion gegebenenfalls einer weiteren Destillation unterzieht.

2. Verfahren nach Anspruch 1, **dadurch gekennzeichnet, dass** man die Reaktionsmischung mit der organischen Base im Verhältnis 1:1,05 bis 1:3, bezogen auf die Reaktionsmischung, umsetzt.

3. Verfahren nach einem der Ansprüche 1 bis 2, **dadurch gekennzeichnet, dass** ein Teil der organischen Base durch Alkalihydroxid ersetzt wird.

4. Verfahren nach einem der Ansprüche 1 bis 3, **dadurch gekennzeichnet, dass** die Leichtsiederfraktion destillativ über eine Kolonne mit 1 bis 20 theoretischen Stufen bei einem Rücklaufverhältnis von 0,1:1 1 bis 10:1 1 abgetrennt wird.

5. Verfahren nach einem der Ansprüche 1 bis 4, **dadurch gekennzeichnet, dass** die Leichtsiederfraktion durch Destillation in eine Monomethylhydrazin (MMH) enthaltende Fraktion und eine die übrigen Nebenprodukte enthaltende Fraktion aufgetrennt wird.

6. Verfahren nach einem der Ansprüche 1 bis 5, **dadurch gekennzeichnet, dass** man die Reaktionsmischung mit der organischen Base bei einer Temperatur im Bereich von 130 bis 170°C umsetzt.

## Claims

1. Process for preparing monomethylhydrazine by methylation of hydrazine or hydrazinium hydrochloride or hydrazinium dihydrochloride with methyl chloride and/or a methanol/HCl mixture, **characterized in that** the reaction mixture formed in the methylation is reacted with an organic base at a pKa greater than 7.0 and a boiling point above 120°C, the organic base being triethanolamine, diethanolamine, monoethanolamine or a mixture thereof, and monomethylhydrazine is removed by distillation from the reaction mixture in a low boiler fraction and this low boiler fraction is optionally subjected to a further distillation.

2. Process according to Claim 1, **characterized in that** the reaction mixture is reacted with the organic base in a ratio of 1:1.05 to 1:3, based on the reaction mixture.

3. Process according to either of Claims 1 and 2, **characterized in that** a portion of the organic base is replaced by alkali metal hydroxide.

4. Process according to one of Claims 1 to 3, **characterized in that** the low boiler fraction is removed by distillation by means of a column having 1 to 20 theoretical plates at a reflux ratio of 0.1:1 to 10:1.

5. Process according to one of Claims 1 to 4, **characterized in that** the low boiler fraction is separated by distillation into a fraction comprising monomethylhydrazine (MMH) and a fraction comprising the remaining by-products.

6. Process according to one of Claims 1 to 5, **characterized in that** the reaction mixture is reacted with the organic base at a temperature in the range of 130 to 170°C.

## Revendications

1. Procédé pour la préparation de monométhylhydrazine par méthylation d'hydrazine ou de chlorhydrate ou de dichlorhydrate d'hydrazinium par du chlorure de méthyle et/ou un mélange de méthanol/HCl, **caractérisé en ce qu'**on transforme le mélange réactionnel formé lors de la méthylation avec une base organique présentant un pKa supérieur à 7,0 et un point d'ébullition supérieur à 120°C, la base organique étant la triéthanolamine, la diéthanolamine, la monoéthanolamine ou un mélange de celles-ci et on sépare la monométhylhydrazine du mélange réactionnel par distillation dans une fraction à bas point d'ébullition et on soumet le cas échéant cette fraction à bas point d'ébullition à une autre distillation.

2. Procédé selon la revendication 1, **caractérisé en ce qu'**on transforme le mélange réactionnel avec la base organique dans un rapport de 1:1,05 à 1:3, par rapport au mélange réactionnel.

3. Procédé selon l'une quelconque des revendications 1 à 2, **caractérisé en ce qu'**une partie de la base organique est remplacée par de l'hydroxyde de métal alcalin.

4. Procédé selon l'une quelconque des revendications 1 à 3, **caractérisé en ce que** la fraction à bas point d'ébullition est séparée par distillation via une colonne présentant 1 à 20 plateaux théoriques à un rapport de reflux de 0,1:1 à 10:1.

5. Procédé selon l'une quelconque des revendications 1 à 4, **caractérisé en ce que** la fraction à bas point d'ébullition est séparée par distillation en une fraction contenant de la monométhylhydrazine (MMH) et une fraction contenant les autres produits secondaires.

6. Procédé selon l'une quelconque des revendications 1 à 5, **caractérisé en ce qu'**on transforme le mélange réactionnel avec la base organique à une température dans la plage de 130 à 170°C.
